# EUROPÄISCHE PATENTSCHRIFT

(11) **EP 1 484 040 B1**
(45) Veröffentlichungstag und Bekanntmachung des Hinweises auf die Patenterteilung: **08.04.2009**
(21) Anmeldenummer: 04012161.8
(22) Anmeldetag: 22.05.2004
(51) Int. Cl.: A61F 5/00, A61B 17/132

(54) **Einrichtung zur Erzeugung einer künstlichen Verengung im Gastro-Intestinal-Trakt**
An apparatus for creating an artificial reduction in the gastrointestinal tract
Dispositif pour générer une constriction artificielle du tract gastro-intestinal

(30) Priorität: 04.06.2003 AT 8632003
(43) Veröffentlichungstag der Anmeldung: 08.12.2004
(73) Patentinhaber: AMI Agency for Medical Innovations GmbH, 6840 Götzis (AT)
(72) Erfinder: Egle, Walter, 6842 Koblach (AT)
(74) Vertreter: Hofmann, Ralf U.

(56) Entgegenhaltungen:
- WO-A-00/09047
- WO-A-01/49245
- US-A1- 2002 198 548
- US-E1- R E36 176

## Beschreibung

Die Erfindung betrifft eine Einrichtung zur Erzeugung einer künstlichen Verengung im Gastro-Intestinal-Trakt mit einem Band, welches ringförmig um den jeweiligen Teil des Gastro-Intestinal-Traktes legbar ist, wobei mittels einer Kolben-Zylinder-Einheit die Größe der Durchlassöffnung des vom Band umgebenen Teils des Gastro-Intestinal-Traktes veränderbar ist und das Band im Bereich seines ersten Endes mit dem Zylinder der Kolben-Zylinder-Einheit verbunden ist.

Eine Einrichtung zur Erzeugung einer künstlichen Verengung im Gastro-Intestinal-Trakt ist beispielsweise in Form eines Magenbandes aus der EP 0 702 529 B1 bekannt. Die Einrichtung weist ein um den Mageneingang legbares Band auf, welches mit einer längs verlaufenden inneren Hohlkammer ausgebildet ist. Zum Verschließen des um den Mageneingang ringförmig gelegten Bandes weist dieses eine Verschlusseinrichtung mit einem am einen Ende des Bandes angeordneten und eine Einstecköffnung aufweisenden ersten Verschlussteil und einem am anderen Ende des Bandes angeordneten, durch die Einstecköffnung einführbaren und gegenüber dieser verrastbaren zweiten Verschlussteil. Zur Verengung des Durchlassquerschnittes der Durchtrittsöffnung des Bandes und somit des Mageneinganges wird die Hohlkammer des Bandes mit einem Füllmedium befüllt, wobei die Menge des Füllmediums vom gewünschten Durchlassquerschnitt abhängt. Zur Befüllung des Bandes mit dem Füllmedium ist ein mit dem Band verbundener Injektionsport vorgesehen, der unter die Haut des Patienten implantiert wird.

Neben einer Ausbildung als Magenband kann eine Einrichtung dieser Art insbesondere auch als Analband zum Verschluss eines, gegebenenfalls künstlichen, Anus ausgebildet sein.

Ein Problem bei solchen Einrichtungen besteht darin, dass diese früher oder später im Laufe ihres Gebrauchs leck werden können, so dass ihre Funktion nicht mehr gegeben ist. Es ist in der Folge eine operative Entfernung und Ersetzung des Bandes erforderlich, was mit einer entsprechenden Belastung des Patienten verbunden ist. Solche Lecks treten in der Praxis insbesondere in der das Band zur Durchtrittsöffnung hin begrenzenden Membran auf. Solche Lecks können beispielsweise durch Materialermüdung im Laufe des Gebrauchs oder durch eine Überfüllung des Bandes auftreten. Für sogenannte "frühe" Lecks, die bis etwa ein Jahr nach dem Einsetzen des Bandes auftreten, sind meist Verletzungen verantwortlich, die bei der operativen, insbesondere laparoskopischen, Platzierung des Bandes durch ein chirurgisches Instrument erfolgt sind.

Eine Einrichtung der eingangs genannten Art ist aus der US 6,470,892 B1 bekannt. Das Band umfasst hierbei einen Kern, der von einem Schlauch umgeben ist. In einem Ausführungsbeispiel ist eine Kolben-Zylinder-Einheit vorgesehen, deren Zylinder am einen Ende des Bandes angebracht ist und deren Kolben über ein Zugseil mit dem Kern verbunden ist, der im Abstand zu diesem Ende des Bandes endet. Durch Verschiebung des Kolbens wird der Kern innerhalb des Schlauches gegenüber diesem verschoben, wodurch das über einen Verschluss ringförmig geschlossene Band zusammengezogen wird. Nachteilig an diesem Band ist unter anderem dessen relativ komplizierter Aufbau.

Aufgabe der Erfindung ist es, eine verbesserte Einrichtung der eingangs genannten Art bereitzustellen, bei welcher die Häufigkeit von operativen Eingriffen nach der Implantation der Einrichtung gering ist. Erfindungsgemäß gelingt dies durch eine Einrichtung mit den Merkmalen des Anspruchs 1.

Bei einer erfindungsgemäßen Einrichtung kann somit der Durchmesser des Bandes mittels der Kolben-Zylinder-Einheit verändert werden. Hierzu wird der Kolben im Zylinderraum des Zylinders verstellt. Beispielsweise kann diese Verstellung durch Zuführen bzw. Entnehmen von Hydraulikflüssigkeit über einen Injektionsport durchgeführt werden. Als Hydraulikflüssigkeit kommt insbesondere steriles Wasser in Frage, eventuell denkbar und möglich wären auch andere körperverträgliche Hydraulikflüssigkeiten.

Weitere Vorteile und Einzelheiten der Erfindung werden im Folgenden anhand des in der beiliegenden Zeichnung dargestellten Ausführungsbeispiels der Erfindung erläutert. In der Zeichnung zeigen:
- Fig. 1: eine Ansicht eines Ausführungsbeispiels einer erfindungsgemäßen Einrichtung;
- Fig. 2: eine Seitenansicht des Ausführungsbeispiels von Fig. 1 und
- Fig. 3: eine Seitenansicht teilweise im Schnitt entlang der Linie AA von Fig. 1.

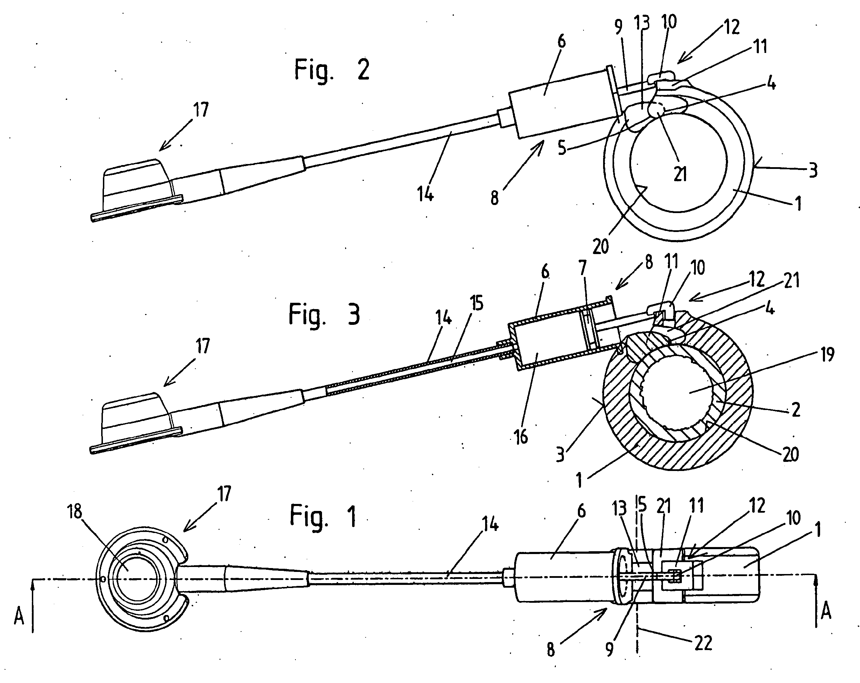

Das in den Fig. dargestellte Ausführungsbeispiel einer erfindungsgemäßen Einrichtung ist als Magenband ausgebildet und weist ein ringförmig um den entsprechenden, in Fig. 3 schematisch dargestellten Teil 2 des Gastro-Intestinal-Traktes, hier den Magen, den Mageneingang oder die Speiseröhre zu legendes Band 1 auf. Das Band 1 besteht beispielsweise, zumindest zum überwiegenden Teil - aus Schaumstoff, vorzugsweise Silikon-Schaumstoff. Im Bereich seiner vom Teil 2 des Gastro-Intestinal-Traktes abgewandten äußeren Oberfläche 3 kann das Band 1 mit einem mit dem Schaumstoffkörper verbundenen verstärkten Rücken, beispielsweise aus massivem Silikon versehen sein, wobei sich dieser verstärkte Rücken über die Länge des Bandes 1 oder zumindest einen Großteil dieser Länge erstrecken kann (nicht dargestellt in den Fig.).

Das Band 1 besitzt ein erstes Ende 4 und ein zweites Ende 5. Im Bereich des ersten Endes 4 des Bandes 1 ist an diesem der Zylinder 6 einer Kolben-Zylinder-Einheit 8 angebracht. Im gezeigten Ausführungsbeispiel ist der Zylinder 6 im Bereich der äußeren Oberfläche 3 des Bandes 1 unlösbar mit diesem verbunden, beispielsweise angeklebt oder teilweise vom Material des Bandes 1 bei dessen Herstellung umspritzt.

Im um den Teil 2 des Gastro-Intestinal-Traktes gelegten Zustand des Bandes 1 besteht weiters eine Verbindung zwischen dem Band 1 im Bereich seines zweiten Endes 5 und dem Kolben 7 der Kolben-Zylinder-Einheit 8. Hierzu ist im gezeigten Ausführungsbeispiel am Kolben 7 eine Kolbenstange 9 angebracht, an deren freiem Ende ein erstes Verschlussglied 10 angeordnet ist, welches in den Zeichnungen schematisch als hakenartiges Element dargestellt ist. Dieses erste Verschlussglied 10 wirkt mit einem am Band 1 im Bereich seines zweiten Endes 5 festgelegten zweiten Verschlussglied 11 zusammen, welches in den Fig. schematisch als plättchenförmiges Teil mit einer Einstecköffnung für das hakenartige erste Verschlussglied 10 dargestellt ist. Die Verschlussglieder 10, 11 bilden zusammen eine Verschlusseinrichtung 12, mittels der das Band im Bereich seines zweiten Endes 5 an der Kolbenstange 9 anbringbar ist. Anstelle der in den Fig. dargestellten Ausbildung dieser Verschlusseinrichtung 12 wären unterschiedliche andere Ausbildungen denkbar und möglich, beispielsweise in der Form Schnappverschlüssen, welche nach dem Verschließen wieder lösbar oder nach dem Verschließen unlösbar ausgebildet sein können.

Wenn in dieser Schrift davon die Rede ist, dass die Verbindung des Bandes mit dem Zylinder 6 bzw. dem Kolben 7 im "Bereich des ersten bzw. zweiten Endes" erfolgt, so bedeutet dies, dass die entsprechenden Verbindungsstellen mit dem Band entweder direkt am entsprechenden Ende des Bandes liegen oder vom jeweiligen Ende 4, 5 des Bandes um weniger als ein Viertel der Längsausdehnung des Bandes 1 beabstandet sind. Vorzugsweise weist zumindest eine dieser Verbindungsstellen einen Abstand vom entsprechenden Ende 4, 5 des Bandes 1 auf, sodass sich ein überstehender Abschnitt 13, 21 ergibt. Im gezeigten Ausführungsbeispiel sind an beiden Enden 4, 5 solche überstehenden Abschnitte 13, 21 angeordnet, die beispielsweise von Endstücken aus ungeschäumtem (massivem) Silikon gebildet werden, die mit dem die restliche Länge des Bandes 1 bildenden Schaumstoffkörper verbunden, z. B. verklebt sind. Im miteinander verbundenen Zustand der beiden Verschlussglieder 10, 11 der Verschlusseinrichtung 12 überlappen an die beiden Enden 4, 5 anschließende Abschnitte des Bandes 1 in Umfangsrichtung gesehen, sodass das Band 1 einen in Seitenansicht gesehen geschlossenen Ring bildet. Je nach der Stellung des Kolbens 7 im Zylinder 6 vergrößert oder verkleinert sich dieser Überlapp, wodurch der Durchmesser des Ringes sich vergrößert oder verkleinert.

Zur Ausbildung dieser Überlappung ist im gezeigten Ausführungsbeispiel eine vom zweiten Ende 5 ausgehende Ausnehmung im Band 1 bzw. überstehenden Abschnitt 21 des Bandes 1 ausgebildet. Der überstehende Abschnitt 13, der das erste Ende 4 bildet, weist eine etwas geringere Breite als die Breite dieser Ausnehmung auf und ragt in diese Ausnehmung je nach Stellung des Kolbens 7 mehr oder weniger weit hinein.

Unterschiedliche andere Ausbildungen einer solchen Überlappung der beiden Endbereiche des Bandes in Umfangsrichtung gesehen sind denkbar und möglich. So könnte beispielsweise das Band 1 im Bereich seiner beiden Enden 4, 5 Abschnitte aufweisen, deren Breite auf etwa den halben Wert des restlichen Bandes verringert ist, wobei diese Abschnitte in Richtung der Achse 22 der Durchgangsöffnung gesehen zueinander versetzt sind und im überlappenden Bereich nebeneinanderliegend angeordnet sind. Auch wäre es denkbar und möglich, dass ein überstehender Abschnitt am einen Ende des Bandes radial innerhalb des anderen Endes des Bandes dieses überlappt und somit an einem an dieses andere Ende des Bandes anschließenden Abschnitt an der inneren Oberfläche 20 des Bandes 1 anliegt. Dieser überstehende und an der inneren Oberfläche 20 des anderen Endabschnittes des Bandes anliegende Abschnitt könnte hierbei etwa keilförmig ausgebildet sein, um am inneren Umfang des Bandes nur eine geringe Stufe auszubilden.

Die Kolben-Zylinder-Einheit 8 steht über ein Röhrchen 14, dessen innerer Kanal 15 mit dem Zylinderraum 16 der Kolben-Zylinder-Einheit 8 kommuniziert, mit einem Injektionsport 17 in Verbindung. Die Einrichtung weist somit ein abgeschlossenes inneres Volumen auf, welches vom Zylinderraum 16, dem Kanal 15 und dem Innenraum des Injektionsports 17 gebildet wird und mit Hydraulikflüssigkeit, beispielsweise sterilem Wasser gefüllt ist. Zur Veränderung der Stellung des Kolbens 7 im Zylinderraum 16 wird die Menge an in diesem abgeschlossenen inneren Volumen vorhandener Hydraulikflüssigkeit geändert. Hierzu besitzt der Injektionsport 18 mit einer von der Nadel einer Spritze durchstechbaren Membran 18. Mittels der durch die Haut des Patienten in die Membran des implantierten Injektionsports eingestochenen Nadel kann Hydraulikflüssigkeit in den Innenraum des Injektionsports 17 eingespritzt oder aus diesem abgezogen werden. Die relativ dick ausgebildete Membran 18 schließt sich nach dem Herausziehen der Nadel der Spritze wiederum dicht. Solche Injektionsports sind bekannt.

Zum Plazieren des Bandes 1 um den Teil 2 des Gastro-Intestinal-Traktes, beispielsweise durch eine laparoskopische Operation, ist das im Bereich des Endes 5 des Bandes 1 angebrachte zweite Verschlussglied 11 zunächst nicht mit dem ersten Verschlussglied 10 verbunden. Mit einem chirurgischen Instrument wird das Band mit seinem zweiten Ende 5 um den Teil 2 des Gastro-Intestinal-Traktes herumgezogen und in der Folge wird das zweite Verschlussglied 11 mit dem an der Kolbenstange 9 angeordneten ersten Verschlussglied 10 verbunden. Das Band 1 umgibt den Teil 2 des Gastro-Intestinal-Traktes nunmehr ringförmig, wie dies in Fig. 3 schematisch dargestellt ist. Zur Verringerung der Größe der Durchlassöffnung 19 des Teils 2 des Gastro-Intestinal-Traktes auf den gewünschten Wert wird das Band 1 mittels der Kolben-Zylinder-Einheit 8 ausgehend von dem in den Fig. dargestellten Zustand entsprechend zusammengezogen, wodurch sich die Endabschnitte des Bandes zunehmend überlappen und der Durchmesser des Bandes sich verringert.

Anstelle der Einbringung oder Abziehung von Hydraulikflüssigkeit in das abgeschlossene innere Volumen der Einrichtung mittels eines Injektionsports der in diesem Sinn eine hydraulische Betätigungseinheit für die Kolben-Zylinder-Einheit 8 bildet, wäre es auch denkbar und möglich, das Röhrchen 14 mit seinem von der Kolben-Zylinder-Einheit 8 abgewandten Ende mit einer hydraulischen Betätigungseinheit zu verbinden, welche ein veränderbares inneres Volumen aufweist. Wenn die Einrichtung als offen- und schließbarer Verschluss ausgebildet ist, beispielsweise als Analband, so müsste die hydraulische Betätigungseinheit in bekannter Weise zwischen zwei Größen ihres inneren Volumens umschaltbar sein, welche dem geschlossenen und geöffneten Zustand der Einrichtung entsprechen.

Unterschiedliche Modifikationen des gezeigten Ausführungsbeispiels der Erfindung sind denkbar und möglich, ohne den Bereich der Erfindung zu verlassen. So könnte beispielsweise auch die Kolbenstange 9 unlösbar mit dem Band 1 im Bereich seines zweiten Endes 5 verbunden sein, beispielsweise angeklebt oder eingespritzt, und der Zylinder 6 könnte über ein am Zylinder 6 angebrachtes erstes Verschlussteil und ein am Band 1 im Bereich seines ersten Endes 4 angebrachtes zweites Verschlussteil mit dem Band 1 verbindbar sein.

Vorteilhafterweise ist die Einrichtung so ausgebildet, dass der dem Teil 2 des Gastro-Intestinal-Traktes zugewandte innere Umfang des Bandes 1 im miteinander verbundenen Zustand der beiden Verschlussglieder 10, 11 im Wesentlichen stufenlos ausgebildet ist oder nur geringe Stufen aufweist, wobei das Material des Bandes 1 im Bereich von gegebenenfalls vorhandenen Stufen (insbesondere im Überlappungsbereich der beiden Endabschnitte) relativ weich ausgebildet ist.

### Legende

### zu den Hinweisziffern:

- 1: Band
- 2: Teil des Gastro-Intestinal-Traktes
- 3: äußere Oberfläche
- 4: erstes Ende
- 5: zweites Ende
- 6: Zylinder
- 7: Kolben
- 8: Kolben-Zylinder-Einheit
- 9: Kolbenstange
- 10: erstes Verschlussglied
- 11: zweites Verschlussglied
- 12: Verschlusseinrichtung
- 13: überstehender Abschnitt
- 14: Röhrchen
- 15: Kanal
- 16: Zylinderraum
- 17: Injektionsport
- 18: Membran
- 19: Durchlassöffnung
- 20: innere Oberfläche
- 21: überstehender Abschnitt
- 22: Achse

## Patentansprüche

1. Einrichtung zur Erzeugung einer künstlichen Verengung im Gastro-Intestinal-Trakt mit einem Band (1), welches ringförmig um den jeweiligen Teil (2) des Gastro-Intestinal-Traktes legbar ist, wobei mittels einer Kolben-Zylinder-Einheit (8) die Größe der Durchlassöffnung (19) des vom Band (1) umgebenen Teils (2) des Gastro-Intestinal-Traktes veränderbar ist und das Band (1) im Bereich seines ersten Endes (4) mit dem Zylinder (6) der Kolben-Zylinder-Einheit (8) verbunden ist, **dadurch gekennzeichnet, dass** das Band (1) im Bereich seines zweiten Endes (5) mit dem Kolben (7) dieser Kolben-Zylinder-Einheit (8) verbunden ist.

2. Einrichtung nach Anspruch 1, **dadurch gekennzeichnet, dass** die Stellung des Kolbens (7) gegenüber dem Zylinder (6) der Kolben-Zylinder-Einheit (8) mittels einer mit der Kolben-Zylinder-Einheit verbundenen hydraulischen Betätigungseinheit (17) veränderbar ist.

3. Einrichtung nach Anspruch 2, **dadurch gekennzeichnet, dass** die hydraulische Betätigungseinheit (17) ein mit dem Zylinderraum (16) der Kolben-Zylinder-Einheit (8) über ein Röhrchen (14) in Verbindung stehender Injektionsport (17) ist.

4. Einrichtung nach einem der Ansprüche 1 bis 3, **dadurch gekennzeichnet, dass** am Kolben (7) eine Kolbenstange (9) angebracht ist, über die der Kolben (7) mit dem Band (1) im Bereich von dessen zweitem Ende (5) in Verbindung steht.

5. Einrichtung nach einem der Ansprüche 1 bis 4, **dadurch gekennzeichnet, dass** zur Verbindung des Bandes (1) mit dem Zylinder (6) der Kolben-Zylinder-Einheit (8) oder zur Verbindung des Bandes (1) mit dem Kolben (7) der Kolben-Zylinder-Einheit ein Verschluss vorhanden ist, der nach dem bei geöffnetem Verschluss durchgeführten Anlegen des Bandes um den jeweiligen Teil (2) des Gastro-Intestinal-Traktes verschließbar ist.

6. Einrichtung nach Anspruch 5, **dadurch gekennzeichnet, dass** zur Verbindung des Bandes (1) im Bereich seines zweiten Endes (5) mit dem Kolben (7) bzw. der am Kolben (7) angebrachten Kolbenstange (9) ein erstes Verschlussglied (10) am Kolben (7) bzw. an der Kolbenstange (9) angeordnet ist, welches mit einem im Bereich des zweiten Endes (5) des Bandes (1) am Band (1) angeordneten zweiten Verschlussglied (11) verbindbar ist.

7. Einrichtung nach einem der Ansprüche 1 bis 6, **dadurch gekennzeichnet, dass** der Zylinder (6) der Kolben-Zylinder-Einheit (8) unlösbar mit dem Band (1) im Bereich seines ersten Endes (4) verbunden ist.

8. Einrichtung nach Anspruch 5, **dadurch gekennzeichnet, dass** zur Verbindung des Bandes (1) im Bereich seines ersten Endes (4) mit dem Zylinder (6) ein erstes Verschlussglied am Zylinder (6) angeordnet ist, welches mit einem im Bereich des ersten Endes (4) des Bandes (1) am Band (1) angeordneten zweiten Verschlussglied verbindbar ist.

9. Einrichtung nach einem der Ansprüche 1 bis 6 oder nach Anspruch 8, **dadurch gekennzeichnet, dass** der Kolben (7) unlösbar mit dem Band (1) im Bereich seines zweiten Endes (5) verbunden ist.

10. Einrichtung nach einem der Ansprüche 1 bis 9, **dadurch gekennzeichnet, dass** das Band (1) zumindest im Wesentlichen aus Silikon besteht.

11. Einrichtung nach einem der Ansprüche 5 bis 10, **dadurch gekennzeichnet, dass** im miteinander verbundenen Zustand der beiden Verschlussglieder (10, 11) an die beiden Enden (4, 5) des Bandes (1) anschließende Abschnitte des Bandes (1) sich in Umfangsrichtung gesehen überlappen, wobei das Band (1) einen in Seitenansicht gesehen geschlossenen Ring bildet.

12. Einrichtung nach Anspruch 11, **dadurch gekennzeichnet, dass** am einen Ende (5) des Bandes eine vom Ende (5) des Bandes ausgehende und in Umfangsrichtung des Bandes (1) verlaufende Ausnehmung ausgebildet ist, in welche ein an das andere Ende (4) des Bandes (1) anschließender Abschnitt des Bandes (1) hineinragt.

13. Einrichtung nach einem der Ansprüche 1 bis 12, **dadurch gekennzeichnet, dass** zumindest im Bereich von einem der Enden (4, 5) des Bandes (1) ein ausgehend von der Verbindungsstelle des Zylinders (6) am Band (1) oder von der Verbindungsstelle des Kolbens (7) am Band (1) sich in Richtung zum entsprechenden Ende (4, 5) hin erstreckender überstehender Abschnitt (13, 21) vorhanden ist.

## Claims

1. A device for the production of an artificial constriction in the gastrointestinal tract, having a band (1) placeable in an annular manner around the specific part (2) of the gastrointestinal tract, wherein the size of the through opening (19) of the part (2) of the gastrointestinal tract surrounded by the band (1) can be altered by means of a piston/cylinder unit (8) and the band (1) is connected in the region of its first end (4) to the cylinder (6) of the piston/cylinder unit (8), **characterised in that** the band (1) is connected in the region of its second end (5) to the piston (7) of this piston/cylinder unit (8).

2. A device according to claim 1, **characterised in that** the position of the piston (7) with respect to the cylinder (6) of the piston/cylinder unit (8) can be altered by means of an hydraulic actuating unit (17) connected to the piston/cylinder unit.

3. A device according to claim 2, **characterised in that** the hydraulic actuating unit (17) is an injection port (17) connected to the cylinder chamber (16) of the piston/cylinder unit (8) via a tube (14).

4. A device according to any one of claims 1 to 3, **characterised in that** a piston rod (9) is attached to the piston (7), and the piston (7) is connected to the band (1) in the region of its second end (5) via the piston rod (9).

5. A device according to any one of claims 1 to 4, **characterised in that** a fastener is present to connect the band (1) to the cylinder (6) of the piston/cylinder unit (8) or to connect the band (1) to the piston (7) of the piston/cylinder unit, which fastener can be closed after the band has been placed around the specific part (2) of the gastrointestinal tract with the fastener in the open state.

6. A device according to claim 5, **characterised in that** a first fastening member (10) is arranged on the piston (7) or on the piston rod (9) to connect the band (1) in the region of its second end (5) to the piston (7) or to the piston rod (9), attached to the piston (7), respectively, which fastening member is connectable to a second fastening member (11) arranged on the band (1) in the region of the second end (5) of the band (1).

7. A device according to any one of claims 1 to 6, **characterised in that** the cylinder (6) of the piston/cylinder unit (8) is connected in an undetachable manner to the band (1) in the region of its first end (4).

8. A device according to claim 5, **characterised in that** a first fastening member is arranged on the cylinder (6) to connect the band (1) in the region of its first end (4) to the cylinder (6), which fastening member is connectable to a second fastening member arranged on the band (1) in the region of the first end (4) of the band (1).

9. A device according to any one of claims 1 to 6 or according to claim 8, **characterised in that** the piston (7) is connected in an undetachable manner to the band (1) in the region of its second end (5).

10. A device according to any one of claims 1 to 9, **characterised in that** the band (1) is at least substantially composed of silicone.

11. A device according to any one of claims 5 to 10, **characterised in that** in the connected-together state of the two fastening members (10, 11), portions of the band (1) adjoining the two ends (4, 5) of the band (1) overlap viewed in the circumferential direction, wherein the band (1) forms a closed ring in lateral view.

12. A device according to claim 11, **characterised in that** an opening issuing from the end (5) of the band and running in the circumferential direction of the band (1) is formed at one end (5) of the band, a portion of the band (1) adjoining the other end (4) of the band (1) projecting into this opening.

13. A device according to any one of claims 1 to 12, **characterised in that** a protruding portion (13, 21) is present in the region of at least one of the ends (4, 5) of the band (1), which portion (13, 21), issuing from the point of connection of the cylinder (6) to the band (1) or from the point of connection of the piston (7) to the band (1), extends towards the corresponding end (4, 5).

## Revendications

1. Installation pour réaliser une constriction artificielle du transit gastro-intestinal comprenant un ruban (1) appliqué sous la forme d'un anneau autour de la partie respective (2) du transit gastro-intestinal,
un ensemble piston-cylindre (8) permet de modifier la taille du passage (19) de la partie (2) du transit gastro-intestinal entouré par le ruban (1), et
le ruban (1) est relié au niveau de sa première extrémité (4) au cylindre (6) de l'ensemble piston-cylindre (8),
**caractérisée en ce que**
le ruban (1) est relié au niveau de sa seconde extrémité (5) au piston (7) de cet ensemble piston-cylindre (8).

2. Installation selon la revendication 1,
**caractérisée en ce que**
la position du piston (7) par rapport au cylindre (6) dans l'ensemble piston-cylindre (8) peut être modifiée à l'aide d'une unité d'actionnement hydraulique (17) reliée à celui-ci.

3. Installation selon la revendication 2,
**caractérisée en ce que**
l'unité d'actionnement hydraulique (17) est un orifice d'injection (18) communiquant avec la chambre (16) du cylindre de l'ensemble piston-cylindre (8) par l'intermédiaire d'un tube (14).

4. Installation selon les revendications 1 à 3,
**caractérisée en ce qu'**
une tige de piston (9), fixée au piston (7), relie ce piston (7) au ruban (1) dans la zone de sa seconde extrémité (5).

5. Installation selon les revendications 1 à 4,
**caractérisée par**
un moyen de fermeture pour relier le ruban (1) au cylindre (6) de l'ensemble piston-cylindre (8) ou pour relier le ruban (1) au piston (7) de l'ensemble piston-cylindre, ce moyen de fermeture étant refermé après qu'en position ouverte, le ruban ait été appliqué autour de la partie respective (2) du transit gastro-intestinal.

6. Installation selon la revendication 5,
**caractérisée en ce que**
pour relier le ruban (1), au niveau de sa seconde extrémité (5), au piston (7) ou à la tige de piston (9) reliée au piston (7), un premier organe de fermeture (10) est prévu sur le piston (7) ou la tige de piston (9), cet organe pouvant être relié à un second organe de fermeture (11) prévu sur le ruban (1) au niveau de sa seconde extrémité (5).

7. Installation selon les revendications 1 à 6,
**caractérisée en ce que**
le cylindre (6) de l'ensemble piston-cylindre (8) est relié de manière solidaire au ruban (1) au niveau de sa première extrémité (4).

8. Installation selon la revendication 5,
**caractérisée en ce qu'**
un premier organe de fermeture est prévu sur le cylindre (6) pour relier le ruban (1) au niveau de sa première extrémité (4) au cylindre (6), ce premier organe de fermeture pouvant être relié à un second organe de fermeture prévu sur le ruban (1) dans sa première zone (4).

9. Installation selon les revendications 1 à 6 ou la revendication 8,
**caractérisée en ce que**
le piston (7) est relié de manière solidaire au ruban (1) au niveau de sa seconde extrémité (5).

10. Installation selon les revendications 1 à 9,
**caractérisée en ce que**
le ruban (1) est au moins principalement en silicone.

11. Installation selon les revendications 5 à 10,
**caractérisée en ce que**
lorsque les deux organes de fermeture (10, 11) aux deux extrémités (4, 5) du ruban (1) sont reliés l'un à l'autre, les segments suivants du ruban (1) se chevauchent dans la direction périphérique, le ruban (1) formant une boucle fermée en vue de côté.

12. Installation selon la revendication 11,
**caractérisée par**
une cavité partant de l'extrémité (5) du ruban (1) et dirigée dans la direction périphérique de celui-ci, cavité dans laquelle pénètre un segment du ruban (1) adjacent à l'autre extrémité (4) du ruban (1).

13. Installation selon les revendications 1 à 12,
**caractérisée en ce qu'**
au moins dans la zone d'une des extrémités (4, 5) du ruban (1), un segment (13, 21) en saillie s'étend à partir du point de liaison du cylindre (6) au ruban (1) ou du point de liaison du piston (7) au ruban (1) en direction de l'autre extrémité correspondante (4 ou 5).
